(19) **Europäisches Patentamt / European Patent Office / Office européen des brevets**

(11) **EP 4 407 629 A1**

(12) **EUROPEAN PATENT APPLICATION**
published in accordance with Art. 153(4) EPC

(43) Date of publication:
**31.07.2024 Bulletin 2024/31**

(21) Application number: **22887675.1**

(22) Date of filing: **28.10.2022**

(51) International Patent Classification (IPC):
*G16H 30/40* (2018.01)   *G16H 30/20* (2018.01)
*G16H 50/50* (2018.01)   *G16H 50/70* (2018.01)
*G06T 7/143* (2017.01)   *G06N 3/08* (2023.01)
*G06N 3/04* (2023.01)

(52) Cooperative Patent Classification (CPC):
**G06N 3/04; G06N 3/08; G06T 7/143; G16H 30/20; G16H 30/40; G16H 50/50; G16H 50/70**

(86) International application number:
**PCT/KR2022/016629**

(87) International publication number:
**WO 2023/075480 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.10.2021 KR 20210145926**
**29.10.2021 KR 20210147228**

(71) Applicant: **Ontact Health Co., Ltd.**
**Seoul 03764 (KR)**

(72) Inventors:
• **SHIM, Hack Joon**
**Seoul 06214 (KR)**
• **JEON, Jae Ik**
**Uijeongbu-si Gyeonggi-do 11742 (KR)**
• **KIM, Se Keun**
**Goyang-si Gyeonggi-do 10306 (KR)**
• **CHOI, Annes**
**Seoul 04193 (KR)**
• **HA, Seong Min**
**Seoul 05343 (KR)**

(74) Representative: **Müller Schupfner & Partner Patent- und Rechtsanwaltspartnerschaft mbB (Muc)**
**Bavariaring 11**
**80336 München (DE)**

(54) **METHOD AND APPARATUS FOR PROVIDING CLINICAL PARAMETER FOR PREDICTED TARGET REGION IN MEDICAL IMAGE, AND METHOD AND APPARATUS FOR SCREENING MEDICAL IMAGE FOR LABELING**

(57)   Provided according to an embodiment of the present invention are a method and apparatus for providing uncertainty data for a predicted target region in a medical image. Also provided according to an embodiment of the present invention are a method and apparatus for screening a medical image for labeling.

100

110

120

IMAGING DEVICE ↔ ELECTRONIC DEVICE

FIG. 1

EP 4 407 629 A1

## Description

### Technical Field

The present invention relates to a method and apparatus for providing a clinical parameter for a predicted target part in a medical image, and a method and apparatus for screening a medical image for labeling.

### Background Art

In general, to determine whether there are diseases in target parts of a subject, medical images of the target parts of the subject are used through radiology tests (e.g., X-ray, ultrasound, computer tomography (CT), angiography, positron emission tomography (PET-CT), or magnetic resonance imaging (MRI), etc.).

By visually checking the acquired medical images and identifying regions of the target parts, specialists can check the target parts and determine whether there are diseases in the target parts.

However, as the number of patients increases, the number of images of the target parts is also doubled, so many experts are required to identify the regions of the target parts and the time it takes for the experts to visually check and identify the target parts one by one also increases.

Accordingly, a method for predicting a target part from an input medical image using an artificial neural network trained to predict a region for the target part based on the medical image has been widely used.

However, since noise exists in the medical image itself due to the performance of an imaging device and/or the movement of the subject, etc., the accuracy and reliability of the predicted results can be lowered even if the artificial neural network is used. Furthermore, if a predictive model is an artificial neural network model trained with a small number of training data, the accuracy and reliability of the predicted results can be further lowered.

Therefore, a method for providing clinical parameters that are substantially the same as clinical parameters of target parts calculated based on results measured by experts is required.

Meanwhile, by visually checking the acquired medical images and identifying the target parts, specialists can check the regions of the target parts and determine whether there are diseases in the target parts.

Since noise exists in the medical image itself due to the performance of the imaging device and/or the movement of the subject, etc., the identification of the region for the target part through the naked eye can result in differences of opinion depending on the skill or experience of the specialists, making it difficult to provide diagnostic results with high reliability and accuracy.

Accordingly, the method for predicting a region of a target part from an input medical image using an artificial neural network trained to predict the region of the target part based on the medical image has been widely used.

However, there is a problem in that the accuracy and reliability of the predicted results are lowered since noise exists in the medical image itself or there is uncertainty about the predicted results of the predictive model due to the small number of training data.

Accordingly, there is a need for a method for improving accuracy and reliability of predicted results in consideration of the above-described uncertainty.

### Detailed Description of the Invention

### Technical Problems

The inventors of the present invention recognized that there is uncertainty about the predicted results using the artificial neural network model due to the noise existing in the medical image itself and/or the insufficient number of training data.

The present invention is directed to providing a method and apparatus for providing a clinical parameter for a predicted target part in a medical image.

Specifically, the problem to be solved by the present invention is to provide a method and apparatus for providing a clinical parameter for a predicted target part in a medical image in order to provide substantially the same results as results predicted by experts using an artificial neural network model.

Problems of the present invention are not limited to the above-described problems. That is, other problems that are not described can be obviously understood by those skilled in the art from the following description.

Meanwhile, the inventors of the present invention recognized that, when a specialist determines a region of a target part by checking a medical image with the naked eye, there is uncertainty about the determined region.

In addition, the inventors of the present invention recognized that, when using the existing predictive model, there is uncertainty about the predicted results if the predictive model is a model trained using a small number of training data.

In addition, the present invention is directed to providing an active learning-based method and apparatus for screening a medical image for labeling.

Specifically, the problem to be solved by the present invention is to provide a method and apparatus for screening a medical image for labeling used for model training to improve performance of an artificial neural network model in consideration of uncertainty about predicted results.

Problems of the present invention are not limited to the above-described problems. That is, other problems that are not described can be obviously understood by those skilled in the art from the following description.


**Technical Solution**

In order to solve the problems described above, there are provided a method and apparatus for providing a clinical parameter for a predicted target part in a medical image.

One aspect of the present invention provides a method for providing a clinical parameter for a predicted target part in a medical image including: acquiring at least one medical image of a target part of a subject from an imaging device; acquiring predicted result data predicting at least one region for the target part from the at least one medical image using a predictive model trained to predict the at least one region corresponding to the target part based on the at least one medical image; calculating the clinical parameter for the target part based on the acquired predicted result data; and providing the calculated clinical parameter.

The predicted result data according to an exemplary embodiment of the present invention can include a plurality of segmented images including a mask region obtained by segmenting each predicted region.

The calculating of the clinical parameter according to an exemplary embodiment of the present invention can mean calculating a median value for a volume of the target part based on a distribution of the mask region for the plurality of segmented images.

The providing of the calculated clinical parameter according to an exemplary embodiment of the present invention can mean providing the calculated median value.

The calculating the clinical parameter according to an exemplary embodiment of the present invention can further include calculating an error range that includes a difference between the median value and a maximum value of the volume of the target part and a difference between the median value and a minimum value of the volume of the target part.

The providing of the calculated clinical parameter according to an exemplary embodiment of the present invention can mean providing the median value and the error range.

The method according to an exemplary embodiment of the present invention can further include providing an image representing the distribution of the mask regions of the plurality of segmented images.

The target part according to an exemplary embodiment of the present invention can include a heart, and the at least one region includes a left atrium, a left ventricle, a right atrium, and a right ventricle.

The predictive model according to an exemplary embodiment of the present invention can be configured to use a probability model trained to calculate a predictive distribution of a model parameter used when predicting at least one region for the target part based on at least one medical image of each of a plurality of recipients.

The predictive model according to an exemplary embodiment of the present invention can be configured so that the predictive distribution of the model parameter calculated by the probability model is reflected in a layer for classifying a class of the at least one region.

The probability model according to an exemplary embodiment of the present invention can be based on a Bayesian neural network.

The predictive model according to an exemplary embodiment of the present invention can be composed of u-net, and a probability distribution of the model parameter can be applied to a last layer of the predictive model.

Another aspect of the present invention provides an apparatus for providing a clinical parameter for a predicted target part in a medical image, including: a communication unit configured to transmit and receive data; and a control unit configured to be connected to the communication unit, in which the control unit is configured to acquire at least one medical image of a target part of a subject from an imaging device through the communication unit, acquire predicted result data predicting at least one region for the target part from the at least one medical image using a predictive model trained to predict the at least one region corresponding to the target part based on the at least one medical image, calculate the clinical parameter for the target part based on the acquired predicted result data, and provide the calculated clinical parameter.

The predicted result data according to an exemplary embodiment of the present invention can include a plurality of segmented images including a mask region obtained by segmenting each predicted region.

The control unit according to an exemplary embodiment of the present invention can be configured to calculate a median value for a volume of the target part based on a distribution of the mask regions of the plurality of segmented images.

The control unit according to an exemplary embodiment of the present invention can be configured to provide the calculated median value as the clinical parameter.

The control unit according to an exemplary embodiment of the present invention can be configured to further calculate an error range that includes a difference between the median value and a maximum value of the volume of the target part and a difference between the median value and a minimum value of the volume of the target part.

The control unit according to an exemplary embodiment of the present invention can be configured to provide the median value and the error range as the clinical parameters.

The control unit according to an exemplary embodiment of the present invention can be configured to further provide an image representing a distribution of the mask regions of the plurality of segmented images.

In order to solve the problems described above, there are provided a method and apparatus for screening a medical image for labeling.

Still another aspect of the present invention provides a method for screening a medical image for labeling, including: acquiring a plurality of medical images of a target part of a subject from an imaging device; acquiring predicted result data representing a result of predicting the at least one region from the plurality of medical images using a predictive model trained to predict at least one region corresponding to the target part based on the plurality of medical images; calculating uncertainty data for the acquired predicted result data; and screening the medical image for labeling from the plurality of medical images based on the calculated uncertainty data.

The uncertainty data according to an exemplary embodiment of the present invention can include aleatoric uncertainty data or include both the aleatoric uncertainty data and epistemic uncertainty data.

The screening of the medical image for labeling according to an exemplary embodiment of the present invention can mean determining a medical image related to the epistemic uncertainty data as the medical image for labeling.

The target part according to an exemplary embodiment of the present invention can include a heart, and the at least one region can include a left atrium, a left ventricle, a right atrium, and a right ventricle.

The predictive model according to an exemplary embodiment of the present invention can be configured to use a predictive distribution of model parameters of a probability model trained to predict the at least one region based on at least one medical image of each of a plurality of recipients.

The probability model according to an exemplary embodiment of the present invention can be based on a Bayesian neural network.

The predictive model according to an exemplary embodiment of the present invention can be configured so that the predictive distribution of the model parameter calculated by the probability model is reflected in a layer for estimating a class of the at least one region.

The predictive model according to an exemplary embodiment of the present invention can be composed of u-net, and a predictive distribution of the model parameter can be applied to a last layer of the predictive model.

The calculating of the uncertainty data according to an exemplary embodiment of the present invention can mean estimating a variance value of the predictive distribution for the uncertainty of the predicted result data.

The estimated variance value according to an exemplary embodiment of the present invention can include a first value for aleatoric uncertainty and a second value for epistemic uncertainty.

The screening of the medical image for labeling according to an exemplary embodiment of the present invention can mean determining at least one medical image whose second value corresponds to a preset first threshold value or greater, as the medical image for labeling.

The screening of the medical image for labeling according to an exemplary embodiment of the present invention can mean determining a medical image whose difference from the maximum value is smaller than a preset second threshold value as the medical image for labeling when at least one medical image corresponding to the first threshold value or greater is sorted from an image with a maximum value to an image with a minimum value.

Still yet another aspect of the present invention provides an apparatus for screening a medical image for labeling, including: a communication unit configured to transmit and receive data; and a control unit configured to be connected to the communication unit, in which the control unit is configured to acquire a plurality of medical images of a target part of a subject from an imaging device through the communication unit, acquire predicted result data representing a result of predicting at least one region from the plurality of medical images using a predictive model trained to predict the at least one region corresponding to the target part based on the plurality of medical images, calculate uncertainty data for the acquired predicted result data, and screen a medical image for labeling from the plurality of medical images based on the calculated uncertainty data.

The control unit according to an exemplary embodiment of the present invention can be configured to determine the medical image including the epistemic uncertainty data as the medical image for labeling.

The control unit according to an exemplary embodiment of the present invention can be configured to estimate a variance value of the predictive distribution for the uncertainty of the predicted result data.

The control unit according to an exemplary embodiment of the present invention can be configured to determine a medical image whose second value corresponds to a preset threshold value or greater, as the medical image for labeling.

The control unit according to an exemplary embodiment of the present invention can be configured to determine a medical image whose difference from the maximum value is less than a preset second threshold value as the medical image for labeling when at least one medical image corresponding to the first threshold value or greater is sorted from an image with a maximum value to an image with a minimum value.

Detailed matters of other exemplary embodiments are described in a detailed description and are illustrated in the accompanying drawings.

**Effects of the Invention**

According to the present invention, by calculating clinical parameters for target parts based on predicted result data that predicts at least one region for the target parts from medical images using an improved predictive model and providing the calculated clinical parameters, even if there is uncertainty about predicted results of an artificial neural network, it is possible to provide clinical parameters with results that are substantially the same as the clinical parameters for the regions of the target parts measured by specialists.

In addition, according to the present invention, by providing an error range that reflects the uncertainty about the predicted results of the artificial neural network in the calculated clinical parameters, it is possible to reduce determination errors by objectively determining the uncertainty about the predicted results.

According to the present invention, by screening an input image for labeling from the input image for the model training based on uncertainty data, it is possible to provide improved predicted results of an active learning-based model.

In addition, according to the present invention, by screening a medical image with high epistemic uncertainty among input images for active learning as the medical image for labeling, labeling the screened medical image, and training the predictive model using the labeled screened medical image as input, it is possible to improve model performance and provide predicted results with improved accuracy and reliability.

In addition, according to the present invention, it is possible to minimize human resources and time required to predict each region for the target part using the predictive model.

The effects according to the present invention are not limited to the contents exemplified above, and further various effects are included in the present specification.

**Brief Description of Drawings**

FIG. 1 is a schematic diagram for describing a system according to an exemplary embodiment of the present invention.
FIG. 2 is a schematic block diagram of an electronic device according to an exemplary embodiment of the present invention.
FIG. 3 is an exemplary diagram illustrating a method for predicting at least one region for a target part using an artificial neural network model according to an exemplary embodiment of the present invention.
FIG. 4 is an exemplary diagram illustrating a predictive model according to an exemplary embodiment of the present invention.
FIG. 5 is a flowchart illustrating a method for providing a clinical parameter for a predicted target part in a medical image according to an exemplary embodiment of the present invention.
FIG. 6 is a flowchart illustrating a method for generating a predictive model using a model parameter of a trained probability model according to an exemplary embodiment of the present invention.
FIG. 7 is an exemplary diagram for describing a method for providing a clinical parameter according to an exemplary embodiment of the present invention.
FIG. 8 is an exemplary diagram illustrating a clinical parameter calculated according to an exemplary embodiment of the present invention and an error range related to the clinical parameter.
FIG. 9 is a schematic diagram for describing a system according to an exemplary embodiment of the present invention.
FIG. 10 is a schematic block diagram of an electronic device according to an exemplary embodiment of the present invention.
FIG. 11 is an exemplary diagram for describing a method for screening a medical image for labeling using an artificial neural network model according to an exemplary embodiment of the present invention.
FIG. 12 is an exemplary diagram illustrating a predictive model according to an exemplary embodiment of the present invention.
FIG. 13 is a flowchart illustrating a method for screening a medical image for labeling according to an exemplary embodiment of the present invention.
FIGS. 14 and 15 are exemplary diagrams illustrating results of quantifying uncertainty of an input image according to an exemplary embodiment of the present invention.

**Best Modes for Carrying out the Invention**

Various advantages and features of the present invention and methods accomplishing them will become apparent from the following description of exemplary embodiments with reference to the accompanying drawings. However, the present invention is not limited to exemplary embodiments to be described below, but can be implemented in various different forms, these exemplary embodiments will be provided only in order to make the present invention complete and allow those skilled in the art to completely recognize the scope of the present invention, Throughout the accompanying drawings, similar components will be denoted by similar reference numerals.

In the present disclosure, an expression "have," "can have," "include," "can include," or the like, indicates existence of a corresponding feature (for example, a numerical value, a function, an operation, a component such as a part, or the like), and does not exclude existence of an additional feature.

In the present disclosure, an expression "A or B," "at least one of A and/or B," "one or more of A and/or B," or the like, can include all possible combinations of items enumerated together. For example, "A or B," "at least one of A and B," or "at least one of A or B" can indicate all of 1) a case in which at least one A is included, 2) a case in which at least one B is included, or 3) a case in which both of at least one A and at least one B are included.

Expressions "first," "second," "1st" or "2nd" or the like, used in the present disclosure can indicate various components regardless of a sequence and/or importance of the components, will be used only in order to distinguish one component from the other components, and do not limit the corresponding components. For example, a first user device and a second user device can indicate different user devices regardless of a sequence or importance thereof. For example, the 'first' component can be named the 'second' component and the 'second' component can also be similarly named the 'first' component, without departing from the scope of the present disclosure.

When it is mentioned that any component (for example: a first component) is (operatively or communicatively) coupled with/to or is connected to another component (for example: a second component), it is to be understood that any component is directly coupled to another component or can be coupled to another component through the other component (for example: a third component). On the other hand, when it is mentioned that any component (for example, a first component) is "directly coupled" or "directly connected" to another component (for example, a second component), it is to be understood that the other component (for example, a third component) is not present between any component and another component.

An expression "~ configured (or set) to" used in the present disclosure can be replaced by an expression "suitable for," "having the capacity to," "~ designed to," "~ adapted to," "- made to," or "~ capable of' depending on a situation. A term "~ configured (or set) to" can not necessarily mean "specifically designed to" in hardware. Instead, an expression "an apparatus configured to -" can mean that the apparatus "is capable of" working with other apparatuses or components. For example, a "processor configured (or set) to perform A, B, and C" can mean a dedicated processor (for example, an embedded processor) for performing the corresponding operations or a generic-purpose processor (for example, a central processing unit (CPU) or an application processor) that can perform the corresponding operations by executing one or more software programs stored in a memory device.

Terms used in the present disclosure can be used only to describe specific exemplary embodiments rather than restricting the scope of other exemplary embodiments. Singular forms can include plural forms unless the context clearly indicates otherwise. All terms used herein including technical and scientific terms have the same meanings as those that are generally understood by those skilled in the art to which the present disclosure pertains. Terms defined in a general dictionary among terms used in the present disclosure can be interpreted as meanings that are the same as or similar to meanings within a context of the related art, and are not interpreted as ideal or excessively formal meanings unless clearly defined in the present disclosure. In some cases, terms can not be interpreted to exclude exemplary embodiments of the present disclosure even though they are defined herein.

Each feature of various exemplary embodiments of the present invention be partially or fully coupled or combined with each other, and as can be fully understood by those skilled in the art, various technical linkages and operations are possible, and each exemplary embodiment can be implemented independently of each other and can be performed together due to the related relationship.

In this specification, an 'image' may be a still image and/or video, but is not limited thereto, and may be a two-dimensional image or a three-dimensional image (i.e., a three-dimensional volume image).

In this specification, 'substantially the same' can mean a case where a difference between two data is smaller than a preset threshold value.

Hereinafter, various exemplary embodiments of the present invention will be described in detail with reference to the accompanying drawings.

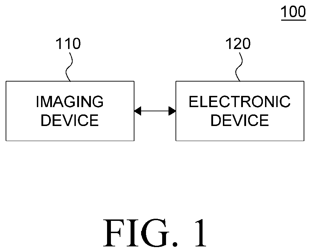

FIG. 1 is a schematic diagram for describing a system according to an exemplary embodiment of the present invention.

Referring to FIG. 1, a system 100 is a system for providing a clinical parameter for a predicted target part in a medical image of a target part of a subject. Here, the medical image is the image of the target part of the subject, and can be an x-ray image, an ultrasonography image, a computer tomography image, an angiography image, a positron emission tomography (PET-CT) image, a magnetic resonance imaging image, etc., but is not limited thereto. The target part is a specific body part of the subject, and can be heart, lungs, bronchi, liver, skin, ureter, urethra, testicles, vagina, anus, laryngeal gland, ovary, thyroid gland, esophagus, nasopharyngeal gland, pituitary gland, salivary gland, prostate, pancreas, adrenal gland, lymph node, spleen, brain, varicose vein, musculoskeletal system, paranasal sinus, spinal cord, kidney, mouth, lip, throat, oral cavity, nasal cavity, small intestine, colon, parathyroid gland, gallbladder, head and neck, breast, bone, bile duct, cervix, heart, and/or hypopharyngeal gland, etc., but is not limited thereto.

First, an imaging device 110 can be an X-ray imaging device, an ultrasound imaging device, a CT imaging device, an angiography imaging device, a positron emission tomography imaging device, and/or an MRI device, etc., to provide at least one medical image of the target part of the subject.

Next, an electronic device 120 can be at least one of a tablet PC, a laptop, and/or a PC, etc. for providing clinical parameters for the target part using predicted result data that predicts at least one region of the target part using at least one medical image provided from the imaging device 110.

Specifically, the electronic device 120 can acquire the predicted result data predicting at least one region of the target part of recipients from at least one medical image, and provide the clinical parameters for the target part based on the acquired predicted result data.

The electronic device 120 can use at least one artificial neural network model or algorithm to acquire the predicted result data predicting at least one region of the target part, but is not limited thereto.

Furthermore, the electronic device 120 may provide the predicted result data predicting a lesion for at least one region of the target part.

In the presented exemplary embodiment, the case where the imaging device 110 and the electronic device 120 are implemented as separate devices has been described, but the present exemplary embodiment is not limited thereto, and the imaging device 110 and the electronic device 120 may be implemented as one device.

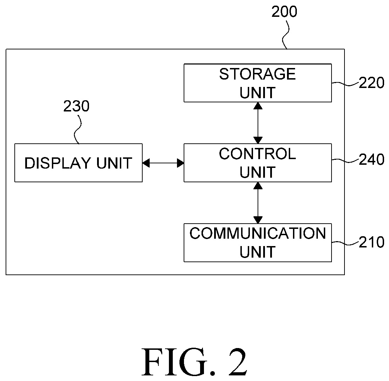

Hereinafter, the electronic device 120 will be described in more detail with reference to FIG. 2.

FIG. 2 is a schematic block diagram of an electronic device according to an exemplary embodiment of the present invention.

Referring to FIG. 2, an electronic device 200 includes a communication unit 210, a storage unit 220, a display unit 230, and a control unit 240. In various exemplary embodiments, the display unit 230 can be optionally provided. In the presented exemplary embodiment, the electronic device 200 can refer to the electronic device 120 of FIG. 1.

The communication unit 210 enables the electronic device 200 to communicate with an external device. The communication unit 210 can be connected to the imaging device 110 using wired/wireless communication and receive at least one medical image of a subject.

The storage unit 220 can acquire predicted result data predicting at least one region of the target part of the subject based on at least one medical image and store various data for providing clinical parameters for the target part based on the acquired predicted results.

In various exemplary embodiments, the storage unit 220 can include at least one type storage medium of a flash memory type storage medium, a hard disk type storage medium, a multimedia card micro type storage medium, a card type memory (for example an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The electronic device 200 may also operate in connection with a web storage performing a storage function of the storage unit 220 on the Internet.

The display unit 230 can display various contents to users. For example, the display unit 230 can display at least one medical image, or the predicted result data and/or clinical parameters.

In various exemplary embodiments, the display unit 230 can include a touch screen, and can receive, for example, touch, gesture, proximity, drag, swipe, hovering input, etc., using an electronic pen or a part of a user's body.

The control unit 240 is operably connected to the communication unit 210, the storage unit 220, and the display unit 230, and can perform various commands to predict at least one region of the target part based on at least one medical image of the subject and provide the clinical parameters for the target part based on the predicted result data for the at least one predicted region.

The control unit 240 can be configured to include at least one of a central processing unit (CPU), a graphics processing unit (GPU), an application processor (AP), a digital signal processing unit (DSP), an arithmetic logic unit (ALU), and an artificial neural network processor (NPU).

Specifically, the control unit 240 acquires at least one medical image of the target part of the subject from the imaging device 110 through the communication unit 210, and acquires the predicted result data that predicts at least one region for the target part from at least one medical image using a predictive model trained to predict at least one region corresponding to the target part based on at least one medical image. Subsequently, the control unit 240 can calculate the clinical parameters for the target part based on the acquired predicted result data and provide the calculated clinical parameters. Here, the predicted result data includes segmented images including a mask region obtained by segmenting each predicted region. Furthermore, the predicted result data may further include at least one of the class of each predicted region and the accuracy for each predicted region.

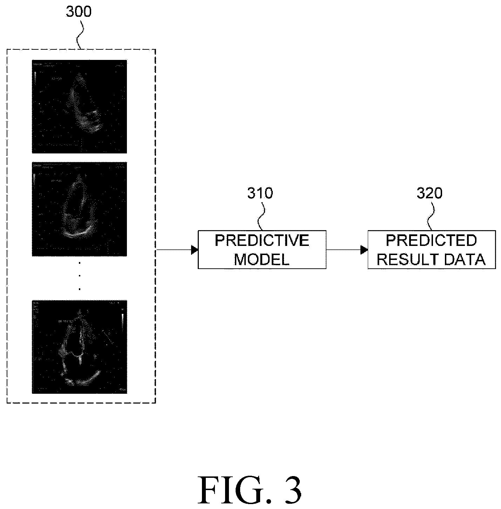

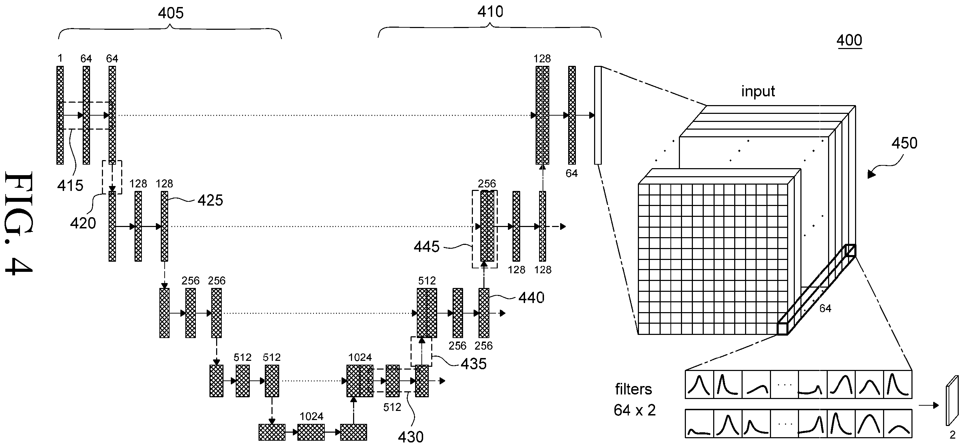

In the following, an operation of the above-described control unit 240 will be described in detail with reference to FIGS. 3 and 4.

FIG. 3 is an exemplary diagram illustrating a method for predicting at least one region for a target part using an artificial neural network model according to an exemplary embodiment of the present invention. In the presented exemplary embodiment, the target part is described as a heart.

Referring to FIG. 3, the control unit 240 can acquire predicted result data 330 predicting a plurality of regions using a predictive model 310 that is trained to predict the plurality of regions corresponding to the heart using a plurality of medical images 300 as input. Here, the plurality of regions corresponding to the heart can be a left atrium, a left ventricle, a right atrium, and a right ventricle.

The predictive model 310 is an artificial neural network model trained to predict the plurality of regions corresponding to the heart based on the plurality of medical images 300, and can be configured to train in advance with a plurality of reference images and predict the plurality of regions from a plurality of newly input medical images 300. Here, the plurality of reference images can be a plurality of medical images of hearts of each of the plurality of recipients. Furthermore, the predictive model 310 may be the artificial neural network model for the image segmentation.

The predictive model 310 can predict each of the plurality of regions corresponding to the hearts from the plurality of input medical images, and output the segmented image including the segmented mask region for each predicted region as the predicted result data.

In general, the medical images can include noise generated by the performance of the imaging device and/or the movement of the subject or target part itself, etc. When the medical images including the noise are input to the predictive model, the predicted results can be inaccurate, so the accuracy and reliability of the corresponding predictive model can be lowered.

In addition, the predicted results of the predictive model can be inaccurate due to the small number of reference images for training the predictive model or the parameters of the model trained with the small number of reference images, so the accuracy and reliability of the corresponding predictive model can also be lowered.

Moreover, the heart is composed of four chambers (left atrium, right atrium, left ventricle, and right ventricle), and a valve performing an appropriate opening and closing function for blood communication is formed between the chambers. The medical images of the heart can have high noise in the valve portion, and when the medical images are used as the input of the predictive model, the predicted results of the predictive model will have high inter/intra variability.

Therefore, the method for providing clinical parameters that are substantially the same as clinical parameters of target parts calculated based on results measured by experts is required.

To this end, the control unit 240 can train a probability model to calculate a predictive distribution of model parameters used when predicting at least one region of the target part based on the plurality of reference images, and use the predictive model 310 that at least partially reflects the predictive distribution of the calculated model parameters.

The probability model for calculating the predictive distribution of the model parameters can be configured based on a Bayesian neural network, but is not limited thereto. For example, a Monte Carlo (MC) dropout method and a neural linear method can be used based on the Bayesian neural network, but it is not limited thereto, and therefore, various methods can be used.

Specifically, the control unit 240 calculates the probability distribution of the model parameters using the Bayesian neural network. When outputting the predicted results that predict at least one region for the target part based on the plurality of reference images used for training, the used Bayesian neural network calculates a posterior predictive distribution of the model parameters representing the predictive distribution of the model parameters used for the at least predicted region. To calculate the posterior predictive distribution, a prior predictive distribution representing the predictive distribution of the model parameters and a likelihood predictive distribution representing the predictive distribution of the predicted results output using the input image and the model parameters are used.

However, since it is generally difficult to calculate the posterior predictive distribution, an approximate value can be obtained through variational inference. In other words, the posterior predictive distribution can be approximated by a variational distribution.

To approximate the posterior predictive distribution of the model parameters, the Bayesian neural network can approximate a variational distribution to the posterior predictive distribution by calculating a difference between the variational distribution and the posterior predictive distribution and minimizing the difference. Here, the difference is called 'Kullback-Leibler divergence.'

As described above, in order to minimize the difference between the variational distribution and the posterior predictive distribution, the Bayesian neural network uses a variational lower bound. The Bayesian neural network can minimize the difference between the variational distribution and the posterior predictive distribution by maximizing the variational lower bound. Here, the variational lower bound means evidence lower bound (ELBO).

The control unit 240 can minimize the difference between the variational distribution and the posterior predictive distribution by maximizing the variational lower bound as described above and thus obtain the approximate value to the posterior predictive distribution, thereby calculating the posterior predictive distribution of the model parameters.

The control unit 240 can generate the predictive model 310 using the calculated posterior predictive distribution.

This will be described in detail with reference to FIG. 4.

FIG. 4 is an exemplary diagram illustrating a predictive model according to an exemplary embodiment of the present invention.

Referring to FIG. 4, a predictive model 400 is configured based on u-net. This predictive model 400 includes a contraction path 405 and an expansion path 410.

The contraction path 405 is based on general convolution networks, and includes one or more layers for a convolution operation, a linear function, and down-sampling for an input value.

The expansion path 410 includes one or more layers for the convolution operation and linear function for up-sampling to expand a size of a feature map.

In the contraction path 405, a 3 × 3 convolution operation is performed a preset number of times at each contraction stage to contract a feature map (415), and the down-sampling operation is performed (420). As a result, the number of channels increases while the size of the feature map decreases (425).

In the expansion path 410, the 3 × 3 convolution operation is performed the preset number of times at each expansion stage to expand the feature map (430), and the upconvolution operation for the up-sampling is performed (435). As a result, the number of channels decreases while the size of the feature map increases (440).

Moreover, the feature map at each expansion stage of the expansion path 410 is merged with the feature map cropped by the size reduced by the convolution operation at each contraction stage of the contraction path 405 at each expansion stage (445).

The last layer of the expansion path 410 is a layer for estimating the information of each channel as a class score, and a filter 450 for acquiring the uncertainty included within all pixels of the medical image is arranged in the layer. The filter 450 reflects the calculated predictive distribution (i.e., posterior predictive distribution) for the model parameters. The last layer can include one or more layers for the 1 × 1 convolution operation and the activation function (sigmoid).

In this way, the predictive model 400 trains the predictive distribution of the model parameters for the plurality of reference images in the last layer, which can have the same effect as outputting the plurality of predicted results using the plurality of predictive models. Referring back to FIG. 3, the control unit 240 can provide the predicted result data 320 that predicts at least one region of the target part using the predictive model 310 that reflects the probability distribution of the model parameters of the probability model.

The predicted result data 320 includes an image including the mask region obtained by segmenting at least one predicted region for the target part. There can be a plurality of such images. As described above, the predictive model 400 can acquire the plurality of predicted results for one reference image, so there can be the plurality of predicted results. Furthermore, the predicted result data can further include at least one of the class of each predicted region and the accuracy for each predicted region.

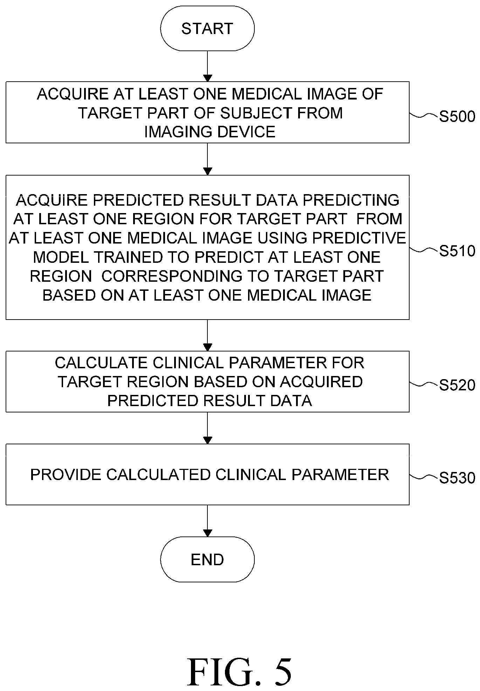

In the following, the method for providing a clinical parameter for a predicted target part in a medical image will be described with reference to FIG. 5.

FIG. 5 is a flowchart for describing a method for providing uncertainty data for a predicted target part in a medical image according to an exemplary embodiment of the present invention.

Referring to FIG. 5, the control unit 240 acquires at least one medical image of the target part of the subject from the imaging device 110 (S500).

The control unit 240 acquires the predicted result data predicting at least one region for the target part from at least one medical image using the predictive model trained to predict the at least one region corresponding to the target part based on the at least one medical image (S510).

The control unit 240 calculates the clinical parameters for the target part based on the acquired predicted result data (S520) and provides the calculated clinical parameters (S530). Here, the predicted result data includes the plurality of segmented images including the mask region obtained by segmenting each predicted region by the predictive model.

To acquire the predicted result data, the control unit 240 uses the probability model trained to calculate the probability distribution of the model parameters used when predicting at least one region for the target part based on at least one medical image of each of the plurality of recipients. The control unit 240 generates the corresponding predictive model so that the probability distribution of the calculated model parameters is reflected in at least part of the predictive model.

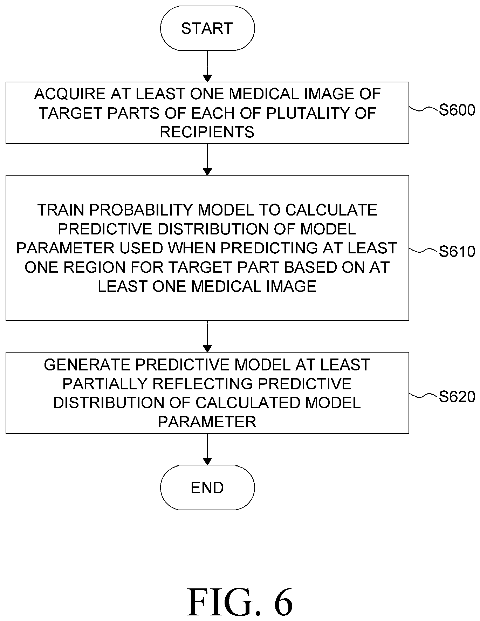

The method for generating a predictive model using a parameter of a trained probability model will be described below with reference to FIG. 6.

FIG. 6 is a flowchart for describing a method for generating a predictive model using a parameter of a trained probability model according to an exemplary embodiment of the present invention.

Referring to FIG. 6, the control unit 240 acquires at least one medical image of the target part for each of the plurality of recipients (S600). Here, at least one medical image for each of the plurality of recipients can refer to the plurality of reference images described above.

The control unit 240 trains the probability model to calculate the predictive distribution of the model parameters used when predicting at least one region of the target part based on at least one medical image (S610).

The control unit 240 generates the predictive model reflecting the predictive distribution of the model parameters calculated by the trained probability model (S620). Specifically, the control unit 240 uses the artificial neural network based on the Bayesian method to calculate the predictive distribution of the model parameters used when predicting at least one region of the target part based on at least one medical image. The control unit 240 generates the predictive model that at least partially reflects the calculated predictive distribution. For example, the control unit 240 can reflect the predictive distribution of the calculated model parameters in the layer related to the class classification of the predictive model.

In this way, the control unit 240 can predict at least one region of the target part and acquire the predicted result data for the at least one predicted region by performing the process described in FIG. 5 using the generated predictive model.

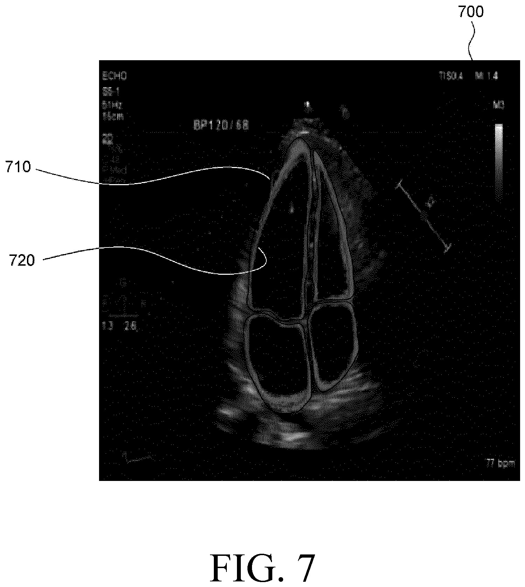

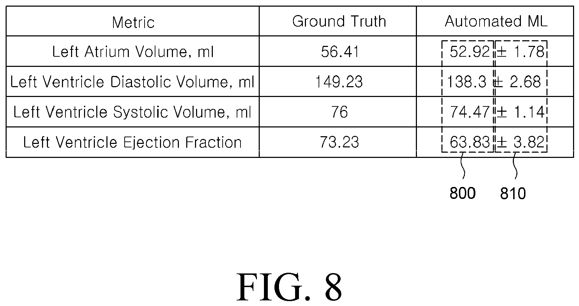

Hereinafter, the clinical parameters for the target part are calculated based on the predicted result data with reference to FIGS. 7 and 8, and the method for providing a calculated clinical parameter will be described.

FIG, 7 is an exemplary diagram illustrating the predicted result data according to an exemplary embodiment of the present invention.

Referring to FIG. 7, the control unit 240 can predict the regions for the left atrium, left ventricle, right atrium, and right ventricle of the heart from the medical images using the predictive model, and provide a segmented image 700 including the mask region obtained by segmenting each predicted region as the predicted result data. The segmented image 700 can be displayed through the display unit 230.

The segmented image 700 can include the distribution of the mask regions of each predicted region by the predictive model. For example, reference numeral 710 can correspond to the largest region among the regions corresponding to the results of predicting the right ventricle, and reference numeral 720 can correspond to the smallest region among the regions corresponding to the results of predicting the right ventricle.

The control unit 240 can further display each region measured by an expert, such as a medical specialist, on the segmented image 700.

The method for providing a clinical parameter for a target part using the segmented image 700 acquired in this way will be described with reference to FIG. 8.

FIG. 8 is an exemplary diagram illustrating the clinical parameters calculated according to an exemplary embodiment of the present invention. In the presented exemplary embodiment, for the same medical image, clinical parameter values calculated based on the results measured by the specialist are compared with clinical parameter values calculated based on the results predicted using the above-described predictive model.

Referring to FIG. 8, when volumes of each of the plurality of mask regions, which are the predicted results for each region of the target part described in FIG. 7, are sorted in order of the largest size, the control unit 240 can calculate a median value 800 located in the very center and provide the calculated median value as the clinical parameter.

Here, the clinical parameters can include, but are not limited to, at least one of the volumes (or sizes) of each region corresponding to the heart, and the contraction/diastolic volumes of each region according to contraction/relaxation of the heart, but these clinical parameters can be determined according to the features of the target part.

Furthermore, the control unit 240 can calculate and provide an error range 810 including a difference between the median value and the maximum value for the volumes of each region and a difference between the median value and the minimum value for the volumes of each region.

Based on this, referring to part of FIG. 8, the median value for volumes of left atrium regions measured by a specialist based on the same medical image can be calculated as '56.41', the median value for the volumes of the left atrium regions predicted by the above-described predictive model can be calculated as '52.92', and the error range can be calculated as '$\pm$1.78'. In addition, the median value for the diastolic volume of the left ventricle regions measured by the specialist can be calculated as '149.23', the median value for the diastolic volumes of the left ventricle regions predicted by the above-described predictive model can be calculated as '138.3', and the error range can be calculated as '$\pm$2.68'.

In this way, the median value 800 calculated based on the predictive model can have substantially the same value as the median value calculated based on the measurement results of the specialist, when considering the error range 810.

Accordingly, the above-described predictive model can provide substantially the same clinical parameters as the clinical parameters calculated based on the results measured by the specialist, even if it includes uncertainty due to noise in the medical image itself and training using the small number of training data.

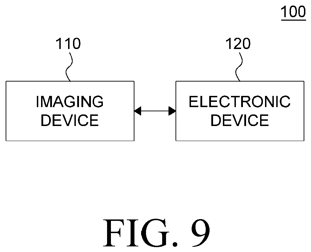

FIG. 9 is a schematic diagram for describing a system according to an exemplary embodiment of the present invention.

Referring to FIG. 9, the system 100 is a system for providing a result of predicting at least one region corresponding to a target part based on a medical image of the target part of the subject. Here, the medical image is the image of the target part of the subject, and can be the x-ray image, the ultrasonography image, the computer tomography (CT) image, the angiography image, the positron emission tomography (PET-CT) image, the magnetic resonance imaging (MRI) image, etc., but is not limited thereto. The target part is a specific body part of the subject, and can be heart, lungs, bronchi, liver, skin, ureter, urethra, testicles, vagina, anus, laryngeal gland, ovary, thyroid gland, esophagus, nasopharyngeal gland, pituitary gland, salivary gland, prostate, pancreas, adrenal gland, lymph node, spleen, brain, varicose vein, musculoskeletal system, paranasal sinus, spinal cord, kidney, mouth, lip, throat, oral cavity, nasal cavity, small intestine, colon, parathyroid gland, gallbladder, head and neck, breast, bone, bile duct, cervix, heart, and/or hypopharyngeal gland, etc., but is not limited thereto.

First, the imaging device 110 can be the X-ray imaging device, the ultrasound imaging device, the CT imaging device, the angiography imaging device, the positron emission tomography imaging device, and/or the MRI device, etc., to provide the plurality of medical images of the target part of the subject.

Next, the electronic device 120 can be at least one of a tablet PC, a laptop, and/or a PC for screening images for labeling from the plurality of medical images provided from the imaging device 110.

Specifically, the electronic device 120 can estimate the uncertainty based on the predicted results of predicting at least one region of the target part of the subject from the plurality of medical images, and screen the medical image for labeling from the plurality of medical images based on the estimated uncertainty.

The electronic device 120 can use at least one artificial neural network model or algorithm to predict at least one region of the target part and acquire the uncertainty data for the predicted results, but is not limited thereto.

Furthermore, the electronic device 120 can predict a lesion for at least one region of the target part and acquire uncertainty data for the predicted lesion, but is not limited thereto.

In the presented exemplary embodiment, the case where the imaging device 110 and the electronic device 120 are implemented as separate devices has been described, but the present exemplary embodiment is not limited thereto, and the imaging device 110 and the electronic device 120 may be implemented as one device.

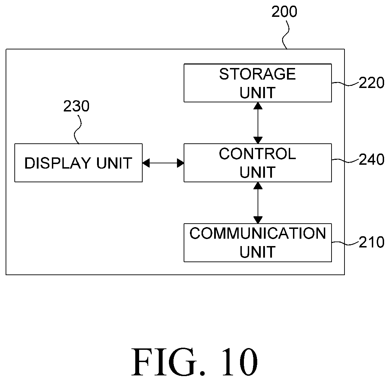

Hereinafter, the electronic device 120 will be described in more detail with reference to FIG. 10.

FIG. 10 is a schematic block diagram of the electronic device according to the exemplary embodiment of the present invention.

Referring to FIG. 10, the electronic device 200 includes the communication unit 210, the storage unit 220, the display unit 230, and the control unit 240. In various exemplary embodiments, the display unit 230 can be optionally provided. In the presented exemplary embodiment, the electronic device 200 can refer to the electronic device 120 of FIG. 9.

The communication unit 210 enables the electronic device 200 to communicate with an external device. The communication unit 210 can be connected to the imaging device 110 using the wired/wireless communication and receive at least one medical image of the subj ect.

The storage unit 220 can store various data for estimating the uncertainty about the results of predicting at least one region of the target part of the subject based on the plurality of medical images, and screening the medical image for labeling based on the estimated uncertainty.

In various exemplary embodiments, the storage unit 220 can include at least one type storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The electronic device 200 may also operate in connection with a web storage performing a storage function of the storage unit 220 on the Internet.

The display unit 230 can display various contents to users. For example, the display unit 230 can display at least one medical image, or display the predicted result data.

In various exemplary embodiments, the display unit 230 can include a touch screen, and can receive, for example, touch, gesture, proximity, drag, swipe, hovering input, etc., using an electronic pen or a part of a user's body.

The control unit 240 is operably connected to the communication unit 210, the storage unit 220, and the display unit 230, and can perform various commands to estimate the uncertainty about the result of predicting at least one region of the target part of the subject based on the plurality of medical images of the subject and screen the medical image for labeling based on the estimated uncertainty.

The control unit 240 can be configured to include at least one of a central processing unit (CPU), a graphics processing unit (GPU), an application processor (AP), a digital signal processing unit (DSP), an arithmetic logic unit (ALU), and an artificial neural network processor (NPU).

Specifically, the control unit 240 acquires at least one medical image of the target part of the subject from the imaging device 110 through the communication unit 210, and acquires the predicted result data that predicts at least one region for the target part from at least one medical image using a predictive model trained to predict at least one region corresponding to the target part based on at least one medical image. Furthermore, the control unit 240 can calculate uncertainty data for the acquired predicted result data and screen the medical image for labeling from the plurality of medical images based on the calculated uncertainty data. Here, the predicted result data can include the segmented image including the mask region obtained by segmenting at least one region predicted by the predictive model, and further include at least one of the class of each predicted region and the accuracy for each predicted region.

In general, the images can include noise generated by the imaging device itself, the movement, etc. When the images including the noise are input to the predictive model, the predicted results can be inaccurate, so the reliability of the corresponding predictive model can be lowered. This can be called the aleatoric uncertainty about prediction.

In addition, the number of reference images for the training of the predictive model can be small, or the predicted results of the predictive model can be inaccurate depending on the model parameters, so the reliability of the predictive model can be lowered. This can be called the epistemic uncertainty about prediction.

Since the noise in the image described above is due to the inherent randomness of the image, the noise can be partially reduced by data pre-processing, but in the case of medical images captured through ultrasound imaging device, CT imaging device, and MRI imaging device, there are limitations in completely removing noise. In particular, the heart is composed of four chambers (left atrium, right atrium, left ventricle, and right ventricle), and a valve performing an appropriate opening and closing function for blood communication is formed between the chambers. Since the thickness of the heart valve is very thin, when imaging the valve, the medical images can have high noise or the valve portion can be blurred.

However, since the epistemic uncertainty about the parameters of the predictive model can be reduced through the collection and training of additional training data, a method for minimizing epistemic uncertainty is required to further improve the accuracy and reliability of the predicted results of the predictive model.

To this end, when all the training data is labeled and the predictive model is trained based on the labeled data, the performance of the predictive model can be greatly improved. However, since the number of training data used is very large, when all of the training data is labeled, for the labeling, a large number of specialized personnel are not only required, but the time and cost will also increase.

Accordingly, there is a need for a method for improving the performance of the predictive model by labeling only a portion of the training data and training the predictive model based on the labeled portion of training data.

To this end, the control unit 240 can estimate uncertainty data for the predicted result data of the predictive model using the active learning method, and screen the medical image for labeling in the plurality of medical images using the epistemic uncertainty data among the estimated uncertainty data.

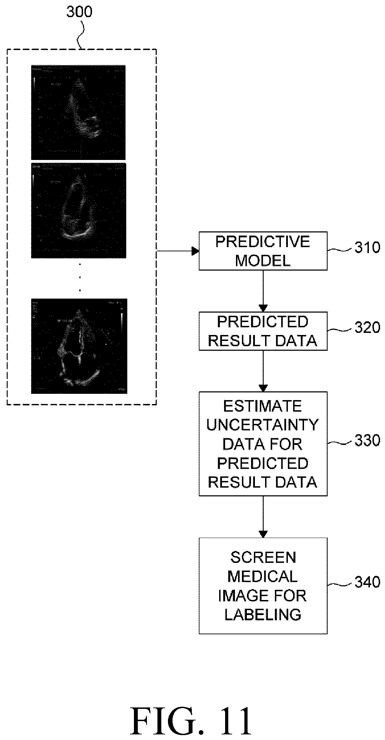

In the following, the operation of the above-described control unit 240 will be described in detail with reference to FIGS. 11 to 15.

FIG. 11 is an exemplary diagram for describing a method for screening a medical image for labeling using an artificial neural network model according to an exemplary embodiment of the present invention. In the presented exemplary embodiment, the target part is described as a heart.

Referring to FIG. 11, the control unit 240 can acquire the predicted result data 320 predicting the plurality of regions using the predictive model 310 that is trained to predict the plurality of regions corresponding to the heart using the plurality of medical images 300 as input. Here, the plurality of regions corresponding to the heart can be a left atrium, a left ventricle, a right atrium, and a right ventricle.

The predictive model 310 is an artificial neural network model trained to predict the plurality of regions corresponding to the heart based on the plurality of medical images 300, and can be configured to train in advance with a plurality of reference images and predict the plurality of regions from the plurality of newly input medical images 300. Here, the plurality of reference images can be a plurality of medical images of hearts of each of the plurality of subjects. Furthermore, the predictive model 310 may be the artificial neural network model for the image segmentation.

As described above, in order for the control unit 240 to determine the medical image for labeling using the epistemic uncertainty data, it is necessary to divide the uncertainty data into the aleatoric uncertainty data and the epistemic uncertainty data.

To this end, the predictive model 310 can at least partially reflect the predictive distribution of the model parameters of the probability model trained to predict at least one region of the target part based on the plurality of reference images.

The probability model for calculating the predictive distribution of the model parameters can be configured based on a Bayesian neural network, but is not limited thereto.

Specifically, when outputting the predicted results that predict at least one region for the target part based on the plurality of reference images used for training, the Bayesian neural network calculates a posterior predictive distribution of the model parameters representing the predictive distribution of the model parameters used for the at least predicted region. To calculate the posterior predictive distribution, the prior predictive distribution representing the predictive distribution of the model parameters and the likelihood predictive distribution representing the predictive distribution of the predicted results output using the input image and the model parameters are used.

However, since it is generally difficult to calculate the posterior predictive distribution, the approximate value can be obtained through the variational inference. In other words, the posterior predictive distribution can be approximated by the variational distribution.

To approximate the posterior predictive distribution of the model parameters, the Bayesian neural network can approximate the variational distribution to the posterior predictive distribution by calculating the difference between the variational distribution and the posterior predictive distribution and minimizing the difference. Here, the difference is called the 'Kullback-Leibler divergence.'

As described above, in order to minimize the difference between the variational distribution and the posterior predictive distribution, the Bayesian neural network uses the variational lower bound. The Bayesian neural network can minimize the difference between the variational distribution and the posterior predictive distribution by maximizing the variational lower bound. Here, the variational lower bound means the evidence lower bound (ELBO).

As described above, the difference between the variational distribution and the posterior predictive distribution can be minimized by maximizing the variational lower bound and thus the approximate value to the posterior predictive distribution can be obtained, so the posterior predictive distribution of the model parameters can be calculated.

The predictive model 310 to which the posterior predictive distribution calculated in this way is applied can be generated.

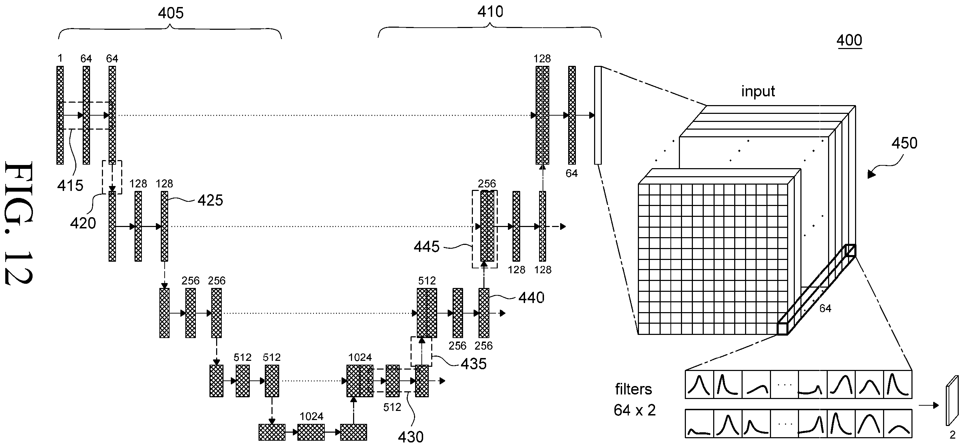

This will be described in detail with reference to FIG. 12.

FIG. 12 is an exemplary diagram illustrating a predictive model according to an exemplary embodiment of the present invention.

Referring to FIG. 12, a predictive model 400 is configured based on u-net. This predictive model 400 includes a contraction path 405 and an expansion path 410.

The contraction path 405 is based on general convolution networks, and includes one or more layers for a convolution operation, a linear function, and down-sampling for an input value.

The expansion path 410 includes one or more layers for the convolution operation and linear function for up-sampling to expand a size of a feature map.

In the contraction path 405, a $3 \times 3$ convolution operation is performed a preset number of times at each contraction stage to contract a feature map (415), and the down-sampling operation is performed (420). As a result, the number of channels increases while the size of the feature map decreases (425).

In the expansion path 410, the $3 \times 3$ convolution operation is performed the preset number of times at each expansion stage to expand the feature map (430), and the upconvolution operation for the up-sampling is performed (435). As a result, the number of channels decreases while the size of the feature map increases (440).

Moreover, the feature map at each expansion stage of the expansion path 410 is merged with the feature map cropped by the size reduced by the convolution operation at each contraction stage of the contraction path 405 at each expansion stage (445).

The last layer of the expansion path 410 is a layer for estimating the information of each channel as a class score, and the filter 450 for acquiring the uncertainty included within all pixels of the medical image is arranged in the layer. The filter 450 reflects the calculated predictive distribution (i.e., posterior predictive distribution) for the model parameters to calculate the uncertainty about each predicted region. The last layer can include one or more layers for the $1 \times 1$ convolution operation and the activation function (sigmoid) to acquire the uncertainty about the predicted results.

Referring back to FIG. 11, the control unit 240 can acquire the predicted result data 320 that predicts at least one region of the target part using the predictive model 310 that reflects the predictive distribution of the model parameters of the probability model (320), and estimate the uncertainty data for the acquired predicted result data (330).

To estimate the uncertainty data, the control unit 240 estimates the probability that the predicted result data is a correct answer using the Bayesian method described above. Specifically, the control unit 240 can estimate the variance of the predictive distribution of the probability that the predicted result data is a correct answer using the following <Equation 1>.

[Equation 1]

$$\mathrm{var}_{q_{\theta}(y^{*}|x)}(y^{*}) \approx \frac{1}{T}\sum_{t=1}^{T}\mathrm{diag}(\hat{p}_t) - \hat{p}_t^{\otimes 2} + \frac{1}{T}\sum_{t=1}^{T}(\hat{p}_t - \bar{p})^{\otimes 2}$$

Here,

$$\frac{1}{T}\sum_{t=1}^{T} \mathrm{diag}(\hat{p}_t) - \hat{p}_t^{\otimes 2}$$

refers to a first value for the aleatoric uncertainty, and

$$\frac{1}{T}\sum_{t=1}^{T} (\hat{p}_t - \bar{p})^{\otimes 2}$$

refers to a second value for the epistemic uncertainty. Then, x denotes the input image, y* denotes a one-hot encoded categorical output, and $q_\theta(y^*|x)$ refers to the variational distribution. In addition, $\hat{p}_t$ refers to the output of the Bayesian neural network, and T means the number of MC samples.

The aleatoric uncertainty data and the epistemic uncertainty data for the predicted result data can be divided through the variance of the estimated predictive distribution.

The control unit 240 screens the medical image for labeling among the plurality of images based on the variance of the predictive distribution estimated by <Equation 1> (340).

Specifically, the control unit 240 can determine, as the medical image for labeling, at least one medical image whose second value among the variance values of the predictive distribution estimated by <Equation 1> corresponds to the preset first threshold value or greater. In other words, the control unit 240 can screen, as the medical image for labeling, the medical images related to the predicted results that have the epistemic uncertainty data of the preset size or more among uncertainty data about results predicted by the predictive model 310 based on the plurality of medical images.

Furthermore, when the second value is sorted from the image with the maximum value to the image with the minimum value among the medical images corresponding to the threshold value or greater, the control unit 240 may determine the medical image whose difference from the maximum value is smaller than the preset second threshold value as the medical image for labeling.

In this way, by screening the medical image with high epistemic uncertainty among the input images for active learning as the medical image for labeling, labeling the screened medical image, and training the predictive model using the labeled screened medical image as input, it is possible to improve the model performance and provide the predicted results with improved accuracy and reliability.

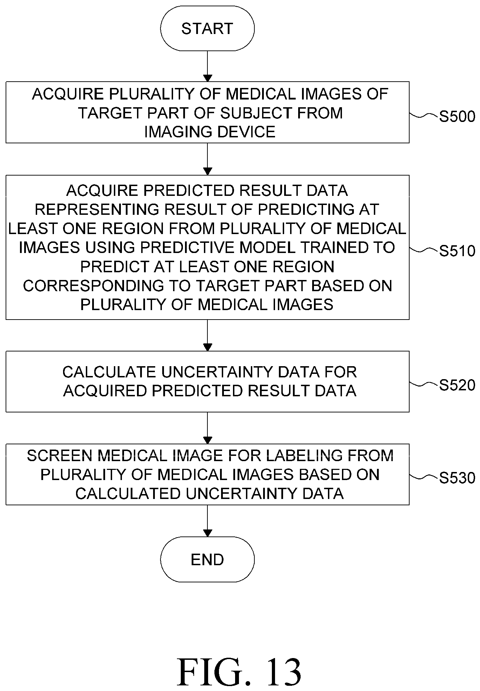

In the following, the method for screening a medical image for labeling based on the predicted results of the predictive model will be described with reference to FIG. 13.

FIG. 13 is a flowchart illustrating a method for screening a medical image for labeling according to an exemplary embodiment of the present invention.

Referring to FIG. 13, the control unit 240 acquires the plurality of medical images of the target part of the subject from the imaging device 110 (S500).

The control unit 240 acquires the predicted result data representing the result of predicting at least one region from the plurality of medical images using the predictive model trained to predict at least one region corresponding to the target part based on the plurality of medical images (S510). The predictive model is composed of the u-net, and configured to use the predictive distribution of the model parameters of the probability model trained to predict at least one region based on at least one medical image of each of the plurality of recipients. For example, the predictive model can be configured so that the predictive distribution of the model parameters of the probability model is reflected in the layer for estimating the class among the plurality of layers constituting the predictive model. The probability model is based on the Bayesian neural network.

Specifically, the control unit 240 acquires, as the predicted result data, the segmented image including the mask region obtained by segmenting each predicted region from the plurality of medical images using the predictive model that reflects the predictive distribution of the model parameters of the trained probability model.

The control unit 240 can calculate the uncertainty data for the acquired predicted result data (S520) and screen the medical image for labeling from the plurality of medical images based on the calculated uncertainty data (S530). Specifically, the control unit 240 can estimate the variance value of the predictive distribution for the uncertainty of the predicted result data, and screen the medical image for labeling using the value related to the epistemic uncertainty among the estimated variance values. For example, the control unit 240 can estimate the variance value of the predictive distribution for the uncertainty using the Bayesian method, and <Equation 1> can be used as described above.

In this way, the control unit 240 can screen the medical image with epistemic uncertainty as the medical image for labeling and train the active learning by labeling the screened medical images.

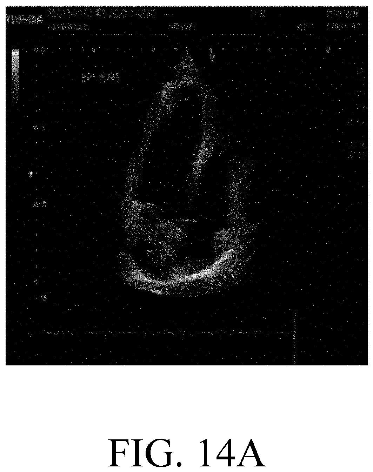

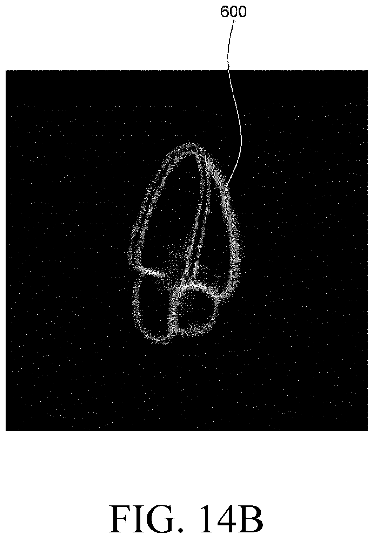

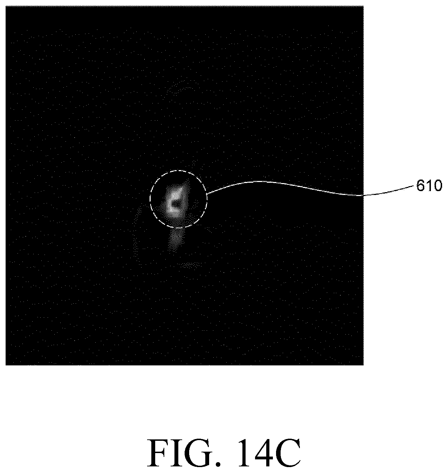

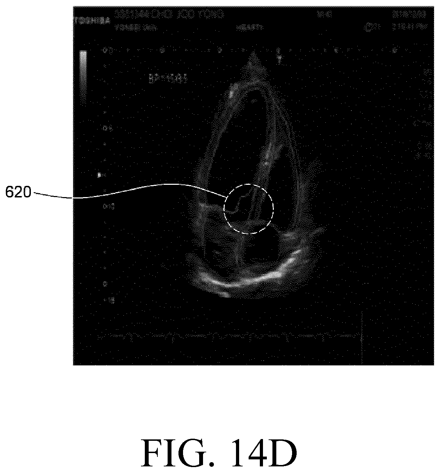

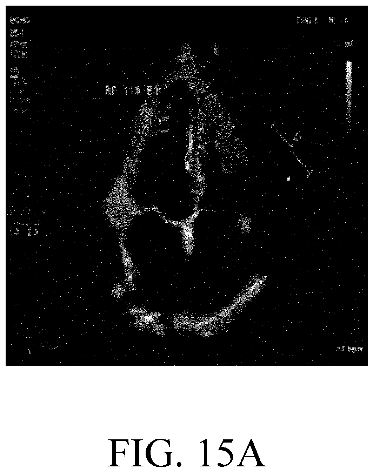

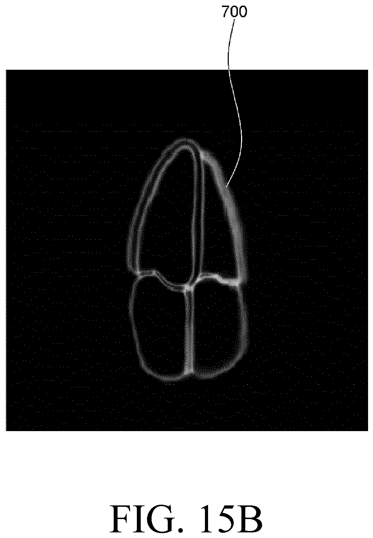

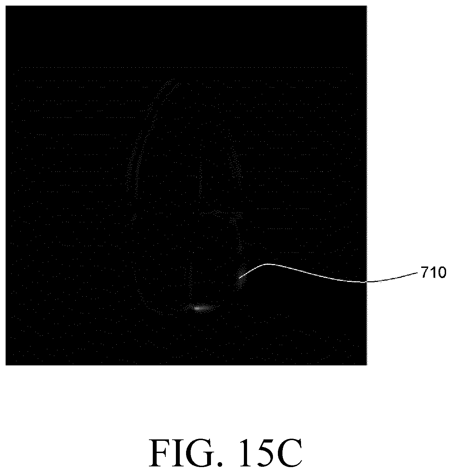

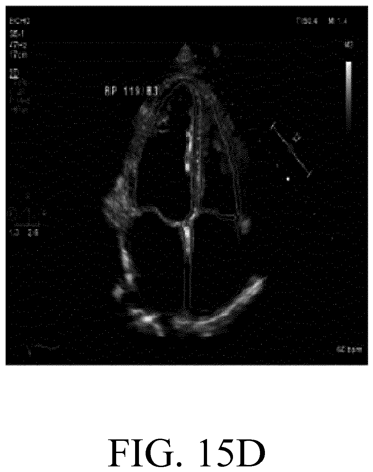

Hereinafter, a method for screening data for labeling in consideration of a size of uncertainty will be described with reference to FIGS. 14 and 15.

FIGS. 14 and 15 are exemplary diagrams illustrating results of quantifying uncertainty of an input image according to an exemplary embodiment of the present invention.

Referring to FIG. 14, (a) is a medical image of a heart region. (a) includes a large amount of noise, so the boundary of the heart region is unclear.

(b) is an image representing the aleatoric uncertainty information of (a) acquired using the above-described <Equation 1>. A boundary portion 600 of the heart region illustrated in (b) is expressed as if smeared by the noise of the image itself. It can be difficult to completely remove this noise.

(c) is an image representing the epistemic uncertainty information of (a) acquired using the above-described <Equation 1>. A portion 610 illustrated in (c) represents the noise due to the uncertainty that can be caused by the small number of training data or the model parameters. The uncertainty of the predictive model can be an important basis for the active learning algorithm.

(d) is the segmented image including the masking region of the heart region predicted by the predictive model.

Specifically, (d) has poor accuracy at the boundary portion of the predicted region due to the aleatoric uncertainty, and has poor accuracy at a portion of reference numeral 620 due to the epistemic uncertainty.

Next, referring to FIG. 15, (a) is the medical image of the heart region. (a) includes a large amount of noise, so the boundary of the heart region is obscure and unclear.

(b) is an image representing the aleatoric uncertainty information of (a) acquired using the above-described <Equation 1>. A boundary portion 700 of the heart region illustrated in (b) is also expressed as if smeared by the noise of the image itself.

(c) is an image representing the epistemic uncertainty information of (a) acquired using the above-described <Equation 1>. (c) has less noise due to the epistemic uncertainty than (c) in FIG. 14. In other words, a portion of reference numeral 710 has a smaller size (or amount) of epistemic uncertainty than that of reference numeral 610 of FIG. 14.

(d) is the segmented image including the masking region of the heart region predicted by the predictive model.

Specifically, (d) illustrates that the accuracy is still low at the boundary portion of the region predicted by the aleatoric uncertainty, but the accuracy due to the epistemic uncertainty is higher than (d) in FIG. 14.

In this way, it is more preferable to screen and label the medical images in order of the medical images with highest uncertainty rather than screening the medical images for labeling with low uncertainty to improve the model performance of the predictive model.

Therefore, the present invention can screen the medical image whose size of the epistemic uncertainty data corresponds to the preset threshold value or greater as the medical image for labeling, label the medical image, and train the predictive model with the labeled medical image as input, thereby improving the model performance and providing the predicted results with improved accuracy and reliability.

The apparatus and the method according to an exemplary embodiment of the present invention can be implemented in a form of program commands that can be executed through various computer means and can be recorded in a computer-readable recording medium. The computer-readable recording medium can include a program command, a data file, a data structure, or the like, alone or a combination thereof.

The program commands recorded in the computer-readable recording medium may be specially designed and constituted for the present invention or be known to those skilled in a field of computer software. Examples of the computer-readable recording medium includes a magnetic medium such as a hard disk, a floppy disk, or a magnetic tape; an optical medium such as a compact disk read only memory (CD-ROM) or a digital versatile disk (DVD); a magneto-optical medium such as a floptical disk; and a hardware device specially configured to store and execute program commands, such as a ROM, a random access memory (RAM), a flash memory, or the like. Examples of the program commands include a high-level language code capable of being executed by a computer using an interpreter, or the like, as well as a machine language code made by a compiler.

The above-mentioned hardware device can be constituted to be operated as one or more software modules in order to perform an operation according to the present invention, and vice versa.

Although the exemplary embodiments of the present invention have been described in more detail with reference to the accompanying drawings, the present invention is not necessarily limited to these exemplary embodiments, but can be variously modified without departing from the scope and spirit of the present invention. Accordingly, exemplary embodiments disclosed in the present invention are not to limit the spirit of the present invention, but are to describe the spirit of the present invention. The scope of the present invention is not limited to these exemplary embodiments.

Therefore, it should be understood that the above-mentioned exemplary embodiments are illustrative in all aspects but are not restrictive. The scope of the present invention should be interpreted by the following claims, and it should be interpreted that all the spirits equivalent to the following claims fall within the scope of the present invention.

[National research and development project supporting the invention]

[Project Identification Number] 1711139017
[Project No.] KMDF_PR_20200901_0156-05
[Ministry name] Multiple ministries
[Project Management (Professional) Organization Name] (Foundation) Korea Medical Device Development Fund
[Research Business Name] Korea Medical Device Development Business (R&D)
[Research Project Name] (Host) Development of 'AI Gatekeeper' solution based on heterogeneous features for screening coronary artery disease
[Contribution Rate] 1/1
[Project Performance Organization Name] Ontact Health Co., Ltd.
[Research Period] November 01, 2020 to December 31, 2023

**Claims**

1. A method for providing a clinical parameter for a predicted target part in a medical image performed by a control unit, the method comprising:

   acquiring at least one medical image of a target part of a subject from an imaging device;
   acquiring predicted result data predicting at least one region for the target part from the at least one medical image using a predictive model trained to predict the at least one region corresponding to the target part based on the at least one medical image;
   calculating the clinical parameter for the target part based on the acquired predicted result data; and
   providing the calculated clinical parameter.

2. The method of claim 1, wherein the predicted result data includes a plurality of segmented images including a mask region obtained by segmenting each predicted region.

3. The method of claim 2, wherein the calculating of the clinical parameter is calculating a median value for a volume of the target part based on a distribution of the mask region for the plurality of segmented images.

4. The method of claim 3, wherein the providing of the calculated clinical parameter is providing the calculated median value.

5. The method of claim 3, wherein the calculating the clinical parameter further includes calculating an error range that includes a difference between the median value and a maximum value of the volume of the target part and a difference between the median value and a minimum value of the volume of the target part.

6. The method of claim 5, wherein the providing of the calculated clinical parameter is providing the median value and the error range.

7. The method of claim 3, further comprising:
   providing an image representing the distribution of the mask region of the plurality of segmented images.

8. The method of claim 1, wherein the target part includes a heart, and the at least one region includes a left atrium, a left ventricle, a right atrium, and a right ventricle.

9. The method of claim 1, wherein the predictive model is configured to use a probability model trained to calculate a predictive distribution of a model parameter used when predicting the at least one region for the target part based on the at least one medical image of each of a plurality of recipients.

10. The method of claim 9, wherein the predictive model is configured so that the predictive distribution of the model parameter calculated by the probability model is reflected in a layer for classifying a class of the at least one region.

11. The method of claim 10, wherein the probability model is based on a Bayesian neural network.

12. The method of claim 10, wherein the predictive model is composed of u-net, and a probability distribution of the model parameter is applied to a last layer of the predictive model.

13. An apparatus for providing a clinical parameter for a predicted target part in a medical image, comprising:

   a communication unit configured to transmit and receive data; and
   a control unit configured to be connected to the communication unit,
   wherein the control unit is configured to acquire at least one medical image of a target part of a subject from an imaging device through the communication unit, acquire predicted result data predicting the at least one region from the at least one medical image using a predictive model trained to predict the at least one region corresponding to the target part based on the at least one medical image, calculate a clinical parameter for the target part based on the acquired predicted result data, and provide the calculated clinical parameter.

14. The apparatus of claim 9, wherein the predicted result data includes a plurality of segmented images including a mask region obtained by segmenting each predicted region.

15. The apparatus of claim 14, wherein the control unit is configured to calculate a median value for a volume of the target part based on a distribution of the mask region of the plurality of segmented images.

16. The apparatus of claim 14, wherein the control unit is configured to provide the calculated median value as the clinical parameter.

17. The apparatus of claim 15, wherein the control unit is configured to further calculate an error range that includes a difference between the median value and a maximum value of the volume of the target part and a difference between the median value and a minimum value of the volume of the target part.

18. The apparatus of claim 16, wherein the control unit is configured to provide the median value and the error range as the clinical parameters.

19. The apparatus of claim 14, wherein the control unit is configured to further provide an image representing a distribution of the mask region of the plurality of segmented images.

20. The apparatus of claim 13, wherein the target part includes a heart, and the at least one region includes a left atrium, a left ventricle, a right atrium, and a right ventricle.

21. The apparatus of claim 13, wherein the predictive model is configured to use a probability model trained to calculate a probability distribution of a model parameter used when predicting the at least one region for the target part based on the at least one medical image of each of a plurality of recipients.

22. The apparatus of claim 21, wherein the predictive model is configured so that the probability distribution of the model parameter calculated by the probability model is reflected in a layer related to class classification.

23. The apparatus of claim 22, wherein the probability model is based on a Bayesian neural network.

24. The apparatus of claim 22, wherein the predictive model is composed of u-net, and a probability distribution of the model parameter is applied to a last layer of the predictive model.

25. A method for screening a medical image for labeling performed by a control unit, comprising:

   acquiring a plurality of medical images of a target part of a subject from an imaging device;
   acquiring predicted result data representing a result of predicting at least one region from the plurality of medical images using a predictive model trained to predict the at least one region corresponding to the target part based on the plurality of medical images;

calculating uncertainty data to the acquired predicted result data; and
screening a medical image for labeling from the plurality of medical images based on the calculated uncertainty data.

26. The method of claim 25, wherein the uncertainty data includes aleatoric uncertainty data, or includes both the aleatoric uncertainty data and epistemic uncertainty data.

27. The method of claim 26, wherein the screening of the medical image for labeling is determining a medical image related to the epistemic uncertainty data as the medical image for labeling.

28. The method of claim 25, wherein the predictive model is configured to use a predictive distribution of model parameters of a probability model trained to predict the at least one region based on at least one medical image of each of a plurality of subjects.

29. The method of claim 28, wherein the probability model is based on a Bayesian neural network.

30. The method of claim 29, wherein the predictive model is configured so that the predictive distribution of the model parameter calculated by the probability model is reflected in a layer for estimating a class of the at least one region.

31. The method of claim 29, wherein the predictive model is composed of u-net, and the predictive distribution of the model parameter is applied to a last layer of the predictive model.

32. The method of claim 29, wherein the calculating of the uncertainty data is estimating a variance value of the predictive distribution for the uncertainty of the predicted result data, the estimated variance value includes a first value for aleatoric uncertainty and a second value for epistemic uncertainty, and the screening of the medical image for labeling is determining the at least one medical image, in which the second value corresponds to a preset first threshold value or greater, as the medical image for labeling.

33. The method of claim 32, wherein the screening of the medical image for labeling is determining a medical image, whose difference from a maximum value is smaller than a preset second threshold value, as the medical image for labeling when at least one medical image corresponding to the first threshold value or greater is sorted from an image with the maximum value to an image with a minimum value.

34. An apparatus for screening a medical image for labeling, comprising:

   a communication unit configured to transmit and receive data; and
   a control unit configured to be connected to the communication unit,
   wherein the control unit is configured to acquire a plurality of medical images of a target part of a subject from an imaging device through the communication unit, acquire predicted result data representing a result of predicting at least one region from the plurality of medical images using a predictive model trained to predict the at least one region corresponding to the target part based on the plurality of medical images, calculate uncertainty data for the acquired predicted result data, and screen a medical image for labeling from the plurality of medical images based on the calculated uncertainty data.

35. The apparatus of claim 34, wherein the uncertainty data includes aleatoric uncertainty data, or includes both the aleatoric uncertainty data and epistemic uncertainty data.

36. The apparatus of claim 35, wherein the control unit is configured to determine the medical image including the epistemic uncertainty data as the medical image for labeling.

37. The apparatus of claim 34, wherein the predictive model is configured to use a predictive distribution of model parameters of a probability model trained to predict the at least one region based on at least one medical image of each of a plurality of subjects.

38. The apparatus of claim 37, wherein the probability model is based on a Bayesian neural network.

39. The apparatus of claim 38, wherein the predictive model is configured so that the predictive distribution of the model parameter calculated by the probability model is reflected in a layer for estimating a class of the at least one region.

**40.** The apparatus of claim 38, wherein the predictive model is composed of u-net, and the predictive distribution of the model parameter is applied to a last layer of the predictive model.

**41.** The apparatus of claim 38, wherein the control unit is configured to estimate a variance value of the predictive distribution for the uncertainty of the predicted result data, the estimated variance value includes a first value for aleatoric uncertainty and a second value for epistemic uncertainty, and the control unit is configured to determine a medical image, in which the second value corresponds to a preset first threshold value or greater, as the medical image for labeling.

**42.** The apparatus of claim 38, wherein the control unit is configured to determine a medical image whose difference from a maximum value is less than a preset second threshold value as the medical image for labeling when at least one medical image corresponding to the first threshold value or greater is sorted from an image with the maximum value to an image with a minimum value.

100

110 120

```
+------------------+        +------------------+
|     IMAGING      | <----> |    ELECTRONIC    |
|     DEVICE       |        |    DEVICE        |
+------------------+        +------------------+
```

# FIG. 1

200

| STORAGE UNIT | 220 |

230

| DISPLAY UNIT | CONTROL UNIT | 240 |

| COMMUNICATION UNIT | 210 |

# FIG. 2

300

310

320

PREDICTIVE
MODEL

PREDICTED
RESULT DATA

FIG. 3

FIG. 4

START

ACQUIRE AT LEAST ONE MEDICAL IMAGE OF TARGET PART OF SUBJECT FROM IMAGING DEVICE — S500

ACQUIRE PREDICTED RESULT DATA PREDICTING AT LEAST ONE REGION FOR TARGET PART FROM AT LEAST ONE MEDICAL IMAGE USING PREDICTIVE MODEL TRAINED TO PREDICT AT LEAST ONE REGION CORRESPONDING TO TARGET PART BASED ON AT LEAST ONE MEDICAL IMAGE — S510

CALCULATE CLINICAL PARAMETER FOR TARGET REGION BASED ON ACQUIRED PREDICTED RESULT DATA — S520

PROVIDE CALCULATED CLINICAL PARAMETER — S530

END

FIG. 5

START

ACQUIRE AT LEAST ONE MEDICAL IMAGE OF
TARGET PARTS OF EACH OF PLUTALITY OF
RECIPIENTS — S600

TRAIN PROBABILITY MODEL TO CALCULATE
PREDICTIVE DISTRIBUTION OF MODEL
PARAMETER USED WHEN PREDICTING AT LEAST
ONE REGION FOR TARGET PART BASED ON AT
LEAST ONE MEDICAL IMAGE — S610

GENERATE PREDICTIVE MODEL AT LEAST
PARTIALLY REFLECTING PREDICTIVE
DISTRIBUTION OF CALCULATED MODEL
PARAMETER — S620

END

# FIG. 6

FIG. 7

| Metric | Ground Truth | Automated ML |
|---|---|---|
| Left Atrium Volume, ml | 56.41 | 52.92 ± 1.78 |
| Left Ventricle Diastolic Volume, ml | 149.23 | 138.3 ± 2.68 |
| Left Ventricle Systolic Volume, ml | 76 | 74.47 ± 1.14 |
| Left Ventricle Ejection Fraction | 73.23 | 63.83 ± 3.82 |

800   810

# FIG. 8

100

110　　　　　　　　120

IMAGING
DEVICE

⟷

ELECTRONIC
DEVICE

# FIG. 9

200

STORAGE
UNIT — 220

230

DISPLAY UNIT ⟷ CONTROL
UNIT — 240

COMMUNICATION
UNIT — 210

# FIG. 10

FIG. 11

FIG. 12

START

ACQUIRE PLURALITY OF MEDICAL IMAGES OF
TARGET PART OF SUBJECT FROM
IMAGING DEVICE — S500

ACQUIRE PREDICTED RESULT DATA
REPRESENTING RESULT OF PREDICTING AT
LEAST ONE REGION FROM PLURALITY OF MEDICAL
IMAGES USING PREDICTIVE MODEL TRAINED TO
PREDICT AT LEAST ONE REGION
CORRESPONDING TO TARGET PART BASED ON
PLURALITY OF MEDICAL IMAGES — S510

CALCULATE UNCERTAINTY DATA FOR
ACQUIRED PREDICTED RESULT DATA — S520

SCREEN MEDICAL IMAGE FOR LABELING FROM
PLURALITY OF MEDICAL IMAGES BASED ON
CALCULATED UNCERTAINTY DATA — S530

END

# FIG. 13

FIG. 14A

600

FIG. 14B

FIG. 14C

FIG. 14D

FIG. 15A

700

FIG. 15B

FIG. 15C

FIG. 15D

<div align="center">

**INTERNATIONAL SEARCH REPORT**

</div>

| | International application No. |
|---|---|
| | **PCT/KR2022/016629** |

**A.    CLASSIFICATION OF SUBJECT MATTER**

**G16H 30/40**(2018.01)i; **G16H 30/20**(2018.01)i; **G16H 50/50**(2018.01)i; **G16H 50/70**(2018.01)i; **G06T 7/143**(2017.01)i; **G06N 3/08**(2006.01)i; **G06N 3/04**(2006.01)i

According to International Patent Classification (IPC) or to both national classification and IPC

**B.    FIELDS SEARCHED**

Minimum documentation searched (classification system followed by classification symbols)

G16H 30/40(2018.01); A61B 5/055(2006.01); G06N 3/02(2006.01); G06N 3/08(2006.01); G06N 99/00(2010.01); G06T 7/10(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심장(heart), 분할(segmentation), 학습(learning), 예측(prediction), 임상 파라미터 (clinical parameter), 불확실성(uncertainty)

**C.    DOCUMENTS CONSIDERED TO BE RELEVANT**

| Category* | Citation of document, with indication, where appropriate, of the relevant passages | Relevant to claim No. |
|---|---|---|
| X | JP 2019-504659 A (ARTERYS INC.) 21 February 2019 (2019-02-21)<br>See paragraphs [0040]-[0106] and figure 5. | 1-24 |
| Y | | 25-42 |
| Y | CN 113555092 A (NVIDIA CORP.) 26 October 2021 (2021-10-26)<br>See paragraphs [0053]-[0077] and claim 2. | 25-42 |
| A | US 2020-0193603 A1 (ARTERYS INC.) 18 June 2020 (2020-06-18)<br>See paragraphs [0028]-[0070] and claims 21-40. | 1-42 |
| A | KR 10-1978317 B1 (INDUSTRY-ACADEMIC COOPERATION FOUNDATION, YONSEI UNIVERSITY) 14 May 2019 (2019-05-14)<br>See paragraphs [0015]-[0024] and claims 1-10. | 1-42 |

☑ Further documents are listed in the continuation of Box C.    ☑ See patent family annex.

| | | |
|---|---|---|
| \* | Special categories of cited documents: | |
| "A" | document defining the general state of the art which is not considered to be of particular relevance | "T"  later document published after the international filing date or priority date and not in conflict with the application but cited to understand the principle or theory underlying the invention |
| "D" | document cited by the applicant in the international application | "X"  document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| **06 February 2023** | **06 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/016629**

**C.     DOCUMENTS CONSIDERED TO BE RELEVANT**

| Category* | Citation of document, with indication, where appropriate, of the relevant passages | Relevant to claim No. |
|---|---|---|
| A | JP 2019-088777 A (PIE MEDICAL IMAGING BV) 13 June 2019 (2019-06-13)<br>See claims 1-17. | 1-42 |

Form PCT/ISA/210 (second sheet) (July 2022)

### INTERNATIONAL SEARCH REPORT
#### Information on patent family members

| International application No. |
| --- |
| **PCT/KR2022/016629** |

| Patent document cited in search report | | | Publication date (day/month/year) | Patent family member(s) | | | Publication date (day/month/year) |
| --- | --- | --- | --- | --- | --- | --- | --- |
| JP | 2019-504659 | A | 21 February 2019 | CN | 106170246 | A | 30 November 2016 |
| | | | | CN | 108291515 | A | 17 July 2018 |
| | | | | CN | 108291515 | B | 31 July 2020 |
| | | | | CN | 108431817 | A | 21 August 2018 |
| | | | | CN | 108601552 | A | 28 September 2018 |
| | | | | CN | 108601552 | B | 09 April 2021 |
| | | | | CN | 108603922 | A | 28 September 2018 |
| | | | | CN | 111894779 | A | 06 November 2020 |
| | | | | EP | 3073915 | A2 | 05 October 2016 |
| | | | | EP | 3073915 | A4 | 16 August 2017 |
| | | | | EP | 3380008 | A2 | 03 October 2018 |
| | | | | EP | 3380008 | B1 | 09 September 2020 |
| | | | | EP | 3380859 | A1 | 03 October 2018 |
| | | | | EP | 3380971 | A1 | 03 October 2018 |
| | | | | EP | 3382194 | A1 | 03 October 2018 |
| | | | | EP | 3382194 | B1 | 27 October 2021 |
| | | | | EP | 3767630 | A1 | 20 January 2021 |
| | | | | EP | 3949837 | A1 | 09 February 2022 |
| | | | | JP | 2017-096212 | A | 01 June 2017 |
| | | | | JP | 2017-506997 | A | 16 March 2017 |
| | | | | JP | 2018-536488 | A | 13 December 2018 |
| | | | | JP | 2018-538050 | A | 27 December 2018 |
| | | | | JP | 2020-163213 | A | 08 October 2020 |
| | | | | JP | 2021-077389 | A | 20 May 2021 |
| | | | | JP | 6411313 | B2 | 24 October 2018 |
| | | | | JP | 6828034 | B2 | 10 February 2021 |
| | | | | JP | 6871250 | B2 | 12 May 2021 |
| | | | | JP | 6993334 | B2 | 13 January 2022 |
| | | | | JP | 7046240 | B2 | 01 April 2022 |
| | | | | KR | 10-2018-0084128 | A | 24 July 2018 |
| | | | | KR | 10-2020-0029620 | A | 18 March 2020 |
| | | | | KR | 10-2096198 | B1 | 01 April 2020 |
| | | | | KR | 10-2443622 | B1 | 14 September 2022 |
| | | | | US | 10117597 | B2 | 06 November 2018 |
| | | | | US | 10331852 | B2 | 25 June 2019 |
| | | | | US | 10398344 | B2 | 03 September 2019 |
| | | | | US | 10718305 | B2 | 21 July 2020 |
| | | | | US | 10869608 | B2 | 22 December 2020 |
| | | | | US | 10871536 | B2 | 22 December 2020 |
| | | | | US | 2016-0338613 | A1 | 24 November 2016 |
| | | | | US | 2017-0076043 | A1 | 16 March 2017 |
| | | | | US | 2018-0256041 | A1 | 13 September 2018 |
| | | | | US | 2018-0256042 | A1 | 13 September 2018 |
| | | | | US | 2018-0259608 | A1 | 13 September 2018 |
| | | | | US | 2018-0335005 | A1 | 22 November 2018 |
| | | | | US | 2019-0069802 | A1 | 07 March 2019 |
| | | | | US | 2019-0272898 | A1 | 05 September 2019 |
| | | | | US | 2020-0054235 | A1 | 20 February 2020 |
| | | | | US | 2020-0375477 | A1 | 03 December 2020 |
| | | | | US | 2021-0085195 | A1 | 25 March 2021 |

Form PCT/ISA/210 (patent family annex) (July 2022)

INTERNATIONAL SEARCH REPORT
Information on patent family members

International application No.

**PCT/KR2022/016629**

| Patent document cited in search report | | | Publication date (day/month/year) | Patent family member(s) | | | Publication date (day/month/year) |
|---|---|---|---|---|---|---|---|
| | | | | WO | 2015-109254 | A2 | 23 July 2015 |
| | | | | WO | 2015-109254 | A3 | 08 October 2015 |
| | | | | WO | 2017-090466 | A1 | 01 June 2017 |
| | | | | WO | 2017-091833 | A1 | 01 June 2017 |
| | | | | WO | 2017-091834 | A1 | 01 June 2017 |
| | | | | WO | 2017-091835 | A2 | 01 June 2017 |
| | | | | WO | 2017-091835 | A3 | 27 July 2017 |
| CN | 113555092 | A | 26 October 2021 | DE | 102021110051 | A1 | 28 October 2021 |
| | | | | GB | 2596201 | A | 22 December 2021 |
| | | | | US | 2021-0334955 | A1 | 28 October 2021 |
| US | 2020-0193603 | A1 | 18 June 2020 | CN | 110475505 | A | 19 November 2019 |
| | | | | CN | 110475505 | B | 05 April 2022 |
| | | | | EP | 3573520 | A2 | 04 December 2019 |
| | | | | EP | 3573520 | A4 | 04 November 2020 |
| | | | | JP | 2020-510463 | A | 09 April 2020 |
| | | | | US | 10600184 | B2 | 24 March 2020 |
| | | | | US | 10902598 | B2 | 26 January 2021 |
| | | | | US | 2018-0218497 | A1 | 02 August 2018 |
| | | | | US | 2018-0218502 | A1 | 02 August 2018 |
| | | | | WO | 2018-140596 | A2 | 02 August 2018 |
| | | | | WO | 2018-140596 | A3 | 07 September 2018 |
| KR | 10-1978317 | B1 | 14 May 2019 | None | | | |
| JP | 2019-088777 | A | 13 June 2019 | EP | 3477324 | A1 | 01 May 2019 |
| | | | | US | 11510587 | B2 | 29 November 2022 |
| | | | | US | 2019-0125206 | A1 | 02 May 2019 |

Form PCT/ISA/210 (patent family annex) (July 2022)